# EUROPÄISCHE PATENTSCHRIFT

(11) **EP 3 999 001 B1**
(45) Veröffentlichungstag und Bekanntmachung des Hinweises auf die Patenterteilung: **20.12.2023**
(21) Anmeldenummer: 20743625.4
(22) Anmeldetag: 15.07.2020
(51) Int. Cl.: A61F 5/02

(54) **ORTHOPÄDIETECHNISCHE EINRICHTUNG**
ORTHOPAEDIC DEVICE
APPAREIL ORTHOPÉDIQUE

(30) Priorität: 19.07.2019 DE 102019119645
(43) Veröffentlichungstag der Anmeldung: 25.05.2022
(73) Patentinhaber: Ottobock SE & Co. KGaA, 37115 Duderstadt (DE)
(72) Erfinder: KREUTEL, Meike, 51067 Köln (DE); MARQUARDT, Charlotte, 21614 Buxtehude (DE); BORNMANN, Jonas, 37115 Duderstadt (DE); TÜTTEMANN, Markus, 45731 Waltrop (DE); VOGEL, Carsten, 37115 Duderstadt (DE); PARTH, Torsten, 63329 Engelsbach (DE); SCHIRRMEISTER, Benjamin, 37077 Göttingen (DE); KLINKERT, Paula, 60439 Frankfurt (DE); MIZERA, Oliver, 37083 Göttingen (DE)
(74) Vertreter: Gramm, Lins & Partner Patent- und Rechtsanwälte PartGmbB
(86) Internationale Anmeldenummer: PCT/EP2020/069984
(87) Internationale Veröffentlichungsnummer: WO 2021/013650

(56) Entgegenhaltungen:
- AU-A1- 2018 256 552
- US-A1- 2017 196 712
- US-A1- 2017 360 588
- US-A1- 2019 015 286

## Beschreibung

Die Erfindung betrifft eine orthopädietechnische Einrichtung zum Unterstützen eines unteren Rückens eines Benutzers, wobei die Einrichtung einen mechanischen Energiespeicher, ein Oberkörperelement mit einem ersten Kraftübertragungselement und ein Oberschenkelelement mit einem zweiten Kraftübertragungselement aufweist. Der mechanische Energiespeicher ist aufladbar und entladbar, indem das Oberschenkelelement relativ zu dem Oberkörperelement verschwenkt wird, wenn das erste Kraftübertragungselement mit dem zweiten Kraftübertragungselement in Eingriff ist.

Derartige Vorrichtungen sind aus dem Stand der Technik seit Langem bekannt und werden insbesondere beim Heben verwendet, um die Person, die beispielsweise einen schweren Gegenstand heben soll, zu unterstützen. Zusätzlich werden derartige Vorrichtungen für Personen verwendet, die in gebückter Haltung arbeiten müssen.

Eine derartige Vorrichtung ist beispielsweise aus der US 443 113 A bekannt. Sie verfügt über Oberschenkelelemente, die an den Oberschenkel des Trägers angeordnet werden. Über Schultergurte wird die Vorrichtung auch am Oberkörper des Trägers angeordnet. Zwischen den Schultergurten und den Oberschenkelelementen befinden sich Blattfederelemente, die beim Bücken gebogen und damit mit potentieller Energie aufgeladen werden. Dadurch üben die Blattfederelemente eine Kraft auf den Oberkörper aus, der die Streckung des Körpers unterstützt. Nachteilig ist jedoch, dass die entstehende Kraft immer ausgeübt wird, wenn ein Winkel zwischen dem Oberkörper und den Oberschenkel verändert wird. Sie wird folglich beispielsweise auch beim Treppensteigen oder Sitzen ausgeübt, was zumindest unbequem, gegebenenfalls jedoch auch störend und unbequem ist.

Prinzipiell ähnliche Vorrichtungen sind beispielsweise aus der US 2017/0196712 A1 und der US 2017/0360588 A1 bekannt. Die den unteren Rücken oder den Oberkörper unterstützende Kraft, die das Aufrichten der Person erleichtern soll, wird jedoch nicht immer ausgeübt. Im erstgenannten Stand der Technik kommt es erst bei Überschreiten eines gewissen Neigungswinkels, wenn also der Winkel zwischen dem Oberkörperelement der Vorrichtung und dem Oberschenkelelement der Vorrichtung einen vorbestimmten Winkel unterschreitet, zur Ausübung der Kraft. Bis zu diesem Winkel kann der Oberkörper relativ zum Oberschenkel geneigt werden, ohne dass ein Aktuator oder Energiespeicher energetisch aufgeladen wird. Dennoch kommt es bei dieser Vorrichtung immer dann zu einer unterstützenden Kraft, wenn der Oberkörper relativ zum Oberschenkel einen Winkel einschließt, der kleiner als der vorbestimmte Grenzwinkel ist, also der Oberkörper relativ zum Oberschenkel geneigt wird.

Aus dem letztgenannten Stand der Technik ist eine Vorrichtung bekannt, bei der die unterstützende Kraft immer dann ausgeübt wird, wenn der Oberkörper relativ zur Vertikalen, also der Richtung, entlang derer die Gewichtskraft wirkt, einen vorbestimmten Winkel einschließt. Damit wird verhindert, dass die Kraft beispielsweise ausgeübt wird, wenn der Träger der Vorrichtung sitzt, sofern dabei der Oberkörper nicht den vorbestimmten Winkel zur Vertikalen überschreitet. Neigt die Person jedoch beim Sitzen den Oberkörper soweit, dass der vorbestimmte Winkel überschritten wird, wird automatisch eine unterstützende Kraft ausgeübt.

Aus dem Stand der Technik sind verschiedene Möglichkeiten bekannt, wie ein Gelenk aus einem aktiven Zustand, in dem beispielsweise eine Bewegung frei möglich ist, in einen passiven oder gebremsten Zustand bringbar ist, in dem beispielsweise die Bewegung nicht mehr oder nur gegen einen Widerstand möglich ist. Die EP 2 645 958 B1 beschreibt beispielsweise ein Gelenk, bei dem dies magnetisch geschaltet wird.

Nachteilig bei allen genannten Richtungen ist, dass nicht sicher eingestellt und erreicht werden kann, dass die Kraft nur dann ausgeübt wird, wenn sie benötigt und gewünscht ist, sondern auch Situationen und Bewegungen auftreten können, bei denen keine unterstützende Kraft benötigt wird oder der untere Rücken nicht beansprucht oder unterstützt werden muss. Der Erfindung liegt daher die Aufgabe zugrunde, eine orthopädietechnische Einrichtung vorzuschlagen, die diese Nachteile aus dem Stand der Technik behebt oder zumindest mildert.

Die Erfindung löst die gestellte Aufgabe durch eine orthopädietechnische Einrichtung zum Unterstützen eines unteren Rückens eines Benutzers gemäß dem Oberbegriff des Anspruchs 1, die sich dadurch auszeichnet, dass die Einrichtung ein Beckenelement aufweist, wobei das Oberschenkelelement um eine erste Schwenkachse schwenkbar an dem Beckenelement angeordnet ist und das Oberkörperelement relativ zu dem Beckenelement bewegbar ist, wobei das erste Kraftübertragungselement mit dem zweiten Kraftübertragungselement in Eingriff und außer Eingriff bringbar ist, indem das Oberkörperelement relativ zu dem Beckenelement bewegt wird.

Der Erfindung liegt die Erkenntnis zugrunde, dass der untere Rücken nicht immer dann einer Unterstützung bedarf, wenn ein Winkel zwischen einem Oberkörperelement, das beispielsweise im Brustbereich oder im Rückenbereich des Oberkörpers des Trägers angeordnet wird, und dem Oberschenkel des Trägers einen vorbestimmten Winkel unterschreitet, die beiden Körperteile also gegeneinander verschwenkt werden. Vielmehr ist eine Unterstützung nur dann notwendig, wenn es zu einer Verschwenkung zwischen dem Oberkörper, also beispielsweise den Brustkorb, des Trägers und dem Becken des Trägers kommt. Durch die erfindungsgemäße Vorrichtung wird erreicht, dass immer dann, wenn diese Verschwenkung zwischen dem Oberkörper und dem Becken des Trägers eintritt, eine unterstützende Kraft ausgeübt wird. Wird hingegen der Oberkörper relativ zum Oberschenkel verschwenkt, ohne dass es zu einer Bewegung des Oberkörpers relativ zum Becken kommt, soll keine Kraft ausgeübt werden.

Das erste Kraftübertragungselement und das zweite Kraftübertragungselement sind dabei derart ausgebildet, dass eine Kraft zwischen den beiden übertragen werden kann, wenn sie sich im Eingriff befinden und keine Kraft übertragen wird, wenn sie nicht in Eingriff sind. Sie sind zudem derart ausgebildet, dass sie mehrfach in Eingriff und außer Eingriff gebracht werden können. Es handelt sich vorzugsweise um zwei Formschlusselemente und/oder zwei Kraftschlusselemente, besonders bevorzugt zwei Reibschlusselemente.

Erfindungsgemäß muss das Oberkörperelement, das vorzugsweise am Rücken oder am Brustkorb des Trägers angeordnet wird, relativ zum Beckenelement, das bevorzugt am Becken des Trägers angeordnet wird, bewegt werden, um das erste Kraftübertragungselement mit dem zweiten Kraftübertragungselement in Eingriff zu bringen. Erst dann kann durch weiteres Verschwenken der Energiespeicher mit mechanischer Energie aufgeladen oder entladen werden. Ohne diese Bewegung des Oberkörperelementes relativ zum Beckenelement bleiben die beiden Kraftübertragungselemente außer Eingriff und eine Bewegung des Oberschenkelelementes relativ zum Oberkörperelement hat keine energetische Aufladung des Energiespeichers zur Folge. Daher kann auch keine die Streckung unterstützende Kraft aufgebracht werden.

Vorzugsweise weist der mechanische Energiespeicher wenigstens ein Federelement auf oder ist ein Federelement. Alternativ oder zusätzlich dazu verfügt er über wenigstens einen Druckspeicher, ein pneumatisches und/oder ein hydraulisches System und/oder über einen hydraulischen Energiespeicher.

Durch die erfindungsgemäße Ausgestaltung der orthopädietechnischen Einrichtung wird folglich erreicht, dass beim Sitzen oder Treppensteigen, wobei es in der Regel nicht zu einer ausreichenden Bewegung zwischen dem Oberkörperelement und dem Beckenelement kommt, keine zusätzliche die Streckung unterstützende Kraft aufgebracht wird, während beispielsweise beim Heben schwerer Gegenstände eine Unterstützung erreicht wird. Bei dieser Art Bewegungen wird das Oberkörperelement relativ zum Beckenelement bewegt und dadurch das erste Kraftübertragungselement mit dem zweiten Kraftübertragungselement in Eingriff gebracht. Wird in diesem Zustand das Oberschenkelelement relativ zum Beckenelement verschwenkt, wird der mechanische Energiespeicher mit potentieller Energie aufgeladen, wodurch eine die Streckung unterstützende Kraft aufgebracht wird.

In einer bevorzugten Ausgestaltung weist das erste Kraftübertragungselement ein Zahnrad auf, das exzentrisch um eine zweite Schwenkachse schwenkbar an dem Beckenelement angeordnet ist. Das zweite Kraftübertragungselement ist in dieser Ausgestaltung vorzugsweise ein Zahnrad, das drehfest am Oberschenkelelement angeordnet ist.

Vorzugsweise ist das Oberkörperelement derart mit dem ersten Kraftübertragungselement und insbesondere mit dem Zahnrad des ersten Kraftübertragungselementes verbunden, dass das erste Kraftübertragungselement um die zweite Schwenkachse verschwenkt wird, wenn das Oberkörperelement relativ zum Beckenelement bewegt wird. Durch diese Bewegung kommt das Zahnrad des ersten Kraftübertragungselementes mit dem zweiten Kraftübertragungselement, das vorzugsweise ebenfalls ein Zahnrad aufweist, in Kontakt und wird mit ihm in Eingriff gebracht. Wird in diesem Zustand der Oberschenkel relativ zum Oberkörper bewegt, beispielsweise indem der Träger in die Knie geht, wird der mechanische Energiespeicher mit potentieller Energie aufgeladen.

Bei der umgekehrten Bewegung wird zunächst diese potentielle Energie wieder abgegeben, indem eine Kraft auf den Oberschenkel und/oder den Oberkörper des Trägers ausgeübt wird, die die Streckung des Körpers des Trägers unterstützt. Erst wenn der Oberkörper und damit das an ihm befestigte Oberkörperelement relativ zum Becken und damit relativ zu dem am Becken befestigten Beckenelement zurück bewegt wird, wird das erste Kraftübertragungselement mit dem zweiten Kraftübertragungselement außer Eingriff gebracht und der mechanische Energiespeicher kann nicht wieder aufgeladen oder weiter entladen werden.

Vorteilhafterweise verfügen das Oberkörperelement und das erste Kraftübertragungselement über zueinander korrespondierende Verbindungselemente, sodass das Oberkörperelement in mehreren Positionen mit dem ersten Kraftübertragungselement verbindbar ist. So kann beispielsweise das Oberkörperelement einen Vorsprung oder Stift oder Pin aufweisen, der in Ausnehmungen oder Vertiefungen am ersten Kraftübertragungselement einsetzbar ist. Selbstverständlich ist auch die umgekehrte Ausgestaltung möglich, bei der das erste Kraftübertragungselement über einen Vorsprung, Stift oder Pin verfügt und die Ausnehmungen und Vertiefungen am Oberkörperelement vorhanden sind. Unabhängig von der tatsächlichen Ausgestaltung ist von Vorteil, wenn das Oberkörperelement und das erste Kraftübertragungselement in unterschiedlichen Positionen und Relativ-Orientierungen zueinander befestigt werden können. Besonders bevorzugt haben diese unterschiedlichen Positionen zur Folge, dass das erste Kraftübertragungselement in unterschiedlichen Winkelstellungen um die zweite Schwenkachse herum positioniert ist. Dadurch kann eingestellt werden, wie stark das Oberkörperelement relativ zum Beckenelement bewegt werden muss, um die beiden Kraftübertragungselemente in Eingriff zu bringen. Es ist folglich einstellbar, zu welchem Zeitpunkt während einer Bewegung der mechanische Energiespeicher aufgeladen werden kann.

Besonders bevorzugt verfügt die orthopädietechnische Einrichtung über eine Verschiebeeinrichtung, die eingerichtet ist, das erste Kraftübertragungselement und/oder das zweite Kraftübertragungselement aufeinander zu zu bewegen, wenn das Oberkörperelement zu dem Beckenelement einen Winkel einnimmt, der kleiner als ein vorbestimmter Grenzwinkel ist. Der Winkel zwischen dem Oberkörperelement und dem Beckenelement beträgt ca. 180°, wenn der Träger der Vorrichtung aufrecht steht. Bückt er sich oder neigt eher den Oberkörper relativ zum Beckenelement, wird dieser Winkel kleiner. Passiert der Winkel dabei den vorbestimmten Grenzwinkel, ist danach der Winkel kleiner als dieser vorbestimmte Grenzwinkel, sodass die Verschiebeeinrichtung die beiden Kraftübertragungselemente aufeinander zu bewegt. Bevorzugt bewegt die Verschiebeeinrichtung entweder das erste Kraftübertragungselement oder das zweite Kraftübertragungselement, während das jeweils andere Kraftübertragungselement ortsfest bleibt. Alternativ dazu bewegt die Verschiebeeinrichtung sowohl das erste Kraftübertragungselement als auch das zweite Kraftübertragungselement.

Im Gegensatz zu der weiter oben beschriebenen Ausführungsform, bei der bei dem Bewegen des Oberkörperelementes relativ zum Beckenelement die beiden Kraftübertragungselemente kontinuierlich aufeinander zu bewegt werden und bei Erreichen des Grenzwinkels miteinander in Eingriff sind, wird bei der hier beschriebenen Ausführungsform zunächst keine Bewegung der beiden Kraftübertragungselemente aufeinander zu erreicht. Erst wenn der Winkel zwischen dem Oberkörperelement und dem Beckenelement den vorbestimmten Grenzwinkel passiert, kommt es zu der hier beschriebenen Bewegung, sodass danach die beiden Kraftübertragungselemente miteinander in Eingriff sind.

Besonders bevorzugt ist die Verschiebeeinrichtung eingerichtet, das erste Kraftübertragungselement und/oder das zweite Kraftübertragungselement voneinander weg zu bewegen, wenn das Oberkörperelement zu dem Beckenelement einen Winkel einnimmt, der größer als der vorbestimmte Grenzwinkel ist.

Um das erste Kraftübertragungselement und/oder das zweite Kraftübertragungselement zu bewegen, ist die Verschiebeeinrichtung eingerichtet, eine Kraft auf das jeweils zu bewegende Kraftübertragungselement auszuüben. Es hat sich als vorteilhaft herausgestellt, wenn diese Kraft aufrechterhalten wird, nachdem das jeweilige Kraftübertragungselement bewegt wurde und die beiden Kraftübertragungselemente in Eingriff oder außer Eingriff gebracht wurden. Damit kann sichergestellt werden, dass es nicht zu einer versehentlichen Veränderung des Zustandes kommt. Sind die beiden Kraftübertragungselemente durch eine Kraft der Verschiebeeinrichtung in Eingriff gebracht worden, so bleibt die dazu verwendete Kraft aufrechterhalten, um zu verhindern, dass die beiden Kraftübertragungselemente versehentlich außer Eingriff gebracht werden, wodurch die Funktionsfähigkeit der orthopädietechnischen Einrichtung beeinträchtigt würde. Gleiches gilt für Kraft, die die beiden Kraftübertragungselemente außer Eingriff bringt. Auch diese Kraft bleibt bevorzugt aufrechterhalten, um zu verhindern, dass es zu einer versehentlichen Verschiebung des jeweiligen Kraftübertragungselementes kommt, wodurch beide Kraftübertragungselemente wieder in Eingriff gebracht würden.

Bevorzugt sind an dem Beckenelement oder an dem Oberschenkelelement wenigstens zwei Magnete und an dem jeweils anderen Element wenigstens ein Magnet derart angeordnet, dass sie eine Kraft aufeinander ausüben, deren Richtung wechselt, wenn der Winkel beim Bewegen des Oberkörperelementes relativ zu dem Beckenelement den vorbestimmten Grenzwinkel passiert. In dieser Ausgestaltung verfügt die Verschiebeeinrichtung folglich über die genannten Magnete. An dem Element am Oberkörperelement oder am Beckenelement, an dem wenigstens zwei Magnete angeordnet sind, sind diese bevorzugt in unterschiedlicher Orientierung angeordnet. Dies bedeutet, dass bei wenigstens einem der Magnete der Nordpol und bei wenigstens einem anderen der Südpol in Richtung auf das jeweils andere Element der orthopädietechnischen Einrichtung gerichtet ist.

Ist der Winkel zwischen dem Oberkörperelement und dem Beckenelement größer als der vorbestimmte Grenzwinkel sind die beiden Kraftübertragungselemente nicht miteinander in Eingriff. Bevorzugt wird folglich eine Kraft ausgeübt, die die beiden Kraftübertragungselemente voneinander weg hält. Dies kann geschehen, indem die Magnete eine Kraft aufeinander ausüben. Dies kann beispielsweise eine abstoßende Kraft sein. Dies wird erreicht, indem ein Magnet des Beckenelementes und ein Magnet des Oberschenkelelementes nahe aneinander positioniert sind, sodass ihre gleichnamigen Pole, also jeweils Südpol oder jeweils Nordpol, aufeinander zu gerichtet sind. Wird nun das Beckenelement relativ zum Oberschenkelelement bewegt, werden auch die an den jeweiligen Elementen angeordneten Magnete bewegt. Es kommt folglich zu einer Verschiebung der bewegt Magnete zueinander. In dem Moment, in dem der Winkel des Oberkörperelementes relativ zum Beckenelement den vorbestimmten Grenzwinkel passiert, kommt vorzugsweise ein zweiter Magnet des Beckenelementes oder des Oberschenkelelementes in den Bereich des wenigstens einen Magnetes des jeweils anderen Elementes. Dabei kommt es nun zu einer anziehenden Kraft, da ungleichnamige Pole der beiden Magnete zueinander gerichtet werden.

Vorteilhafterweise ist die orthopädietechnische Einrichtung in einen aktiven und in einen passiven Zustand bringbar. Der aktive Zustand wurde bisher beschrieben und zeichnet sich dadurch aus, dass das erste Kraftübertragungselement und das zweite Kraftübertragungselement in Eingriff und außer Eingriff bringbar sind, indem das Oberkörperelement relativ zu dem Beckenelement bewegt wird. Im passiven Zustand ist dies nicht möglich. Wird im passiven Zustand das Oberkörperelement relativ zu dem Beckenelement bewegt, kommen die beiden Kraftübertragungselemente nicht in Eingriff oder außer Eingriff.

Vorzugsweise verfügt die Einrichtung über wenigstens ein Betätigungselement, durch dessen Betätigung die Einrichtung von dem aktiven Zustand in den passiven Zustand und/oder umgekehrt bringbar ist. Durch ein solches Betätigungselement kann beispielsweise eine Bewegung des ersten Kraftübertragungselementes von der Bewegung des Oberkörperelementes abgekoppelt werden. Durch erneute Betätigung des Betätigungselementes wird die Bewegung wieder gekoppelt, sodass die Kraftübertragungselemente in Eingriff bringbar sind.

Vorzugsweise verfügt der mechanische Energiespeicher über wenigstens ein Federelement, vorzugsweise eine Spiralfeder.

Mit Hilfe der beiliegenden Zeichnungen werden nachfolgend einige Ausführungsbeispiele der vorliegenden Erfindung näher erläutert. Es zeigen:
- Figur 1 -: eine orthopädietechnische Einrichtung gemäß einem ersten Ausführungsbeispiel der vorliegenden Erfindung im angelegten Zustand,
- Figur 2 und 3 -: einen Teil der Einrichtung in unterschiedlichen Ansichten,
- Figur 4 -: den Teil aus den Figuren 2 und 3 in einer Explosionsdarstellung,
- Figur 5 -: die Darstellung des Teils in einem teilweisen demontierten Zustand,
- Figuren 6 bis 8 -: Darstellungen eines Teils einer weiteren Ausführungsform der vorliegenden Erfindung,
- Figuren 9 und 10 -: schematische Darstellungen eines Teils einer weiteren Ausführungsform der vorliegenden Erfindung,
- Figuren 11 und 12 -: schematische Darstellungen eines Teils einer weiteren Ausführungsform der vorliegenden Erfindung und
- Figuren 13 bis 15 -: Darstellungen einer Ausführungsform der Erfindung im angelegten Zustand.

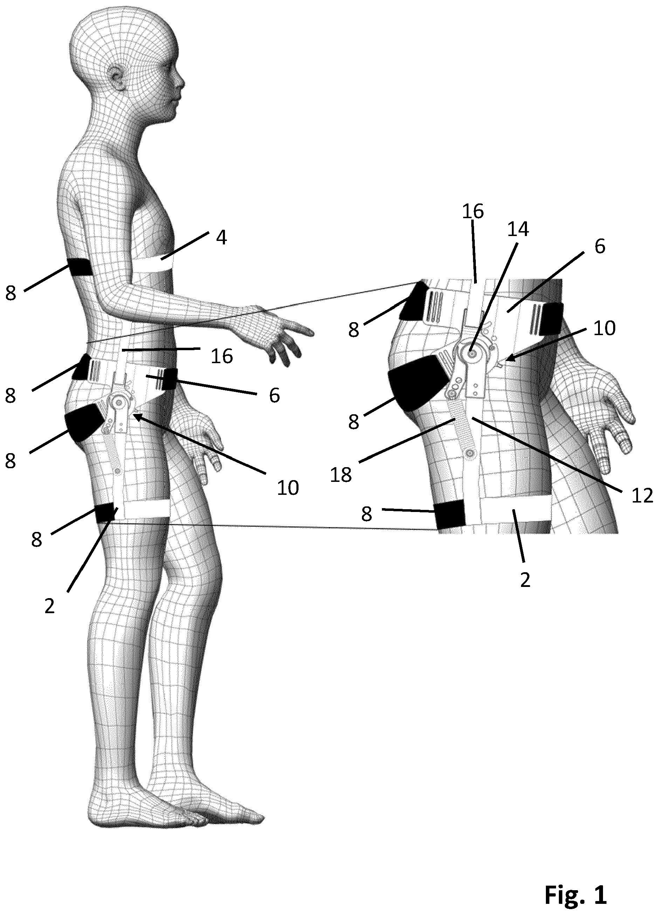

Figur 1 zeigt die orthopädietechnische Einrichtung in einem angelegten Zustand. Sie verfügt über ein Oberschenkelelement 2, das an einem Oberschenkel des Benutzers angeordnet ist, und über ein Oberkörperelement 4, das am Oberkörper angeordnet ist. Die Vorrichtung verfügt zudem über ein Beckenelement 6, das am Becken des Benutzers angeordnet ist. Sowohl das Beckenelement 6 als auch das Oberschenkelelement 2 und das Oberkörperelement 4 sind am jeweiligen Körperteil des Benutzers angeordnet.

Die orthopädietechnische Einrichtung verfügt zudem über eine Gelenkeinrichtung 10, die im gezeigten Ausführungsbeispiel mehrere Aufgaben erfüllt. Einerseits ist das Oberschenkelelement 2 über eine erste Schiene 12 um eine erste Schwenkachse 14 an dem Beckenelement 6 angeordnet. Über eine zweite Schiene 16 ist auch das Oberkörperelement 4 schwenkbar am Beckenelement 6 angeordnet, wobei die Schwenkachse in diesem Ausführungsbeispiel mit der ersten Schwenkachse 14 zusammenfällt.

Die orthopädietechnische Einrichtung verfügt zudem über einen mechanischen Energiespeicher 18, der im gezeigten Ausführungsbeispiel eine Zugfeder ist.

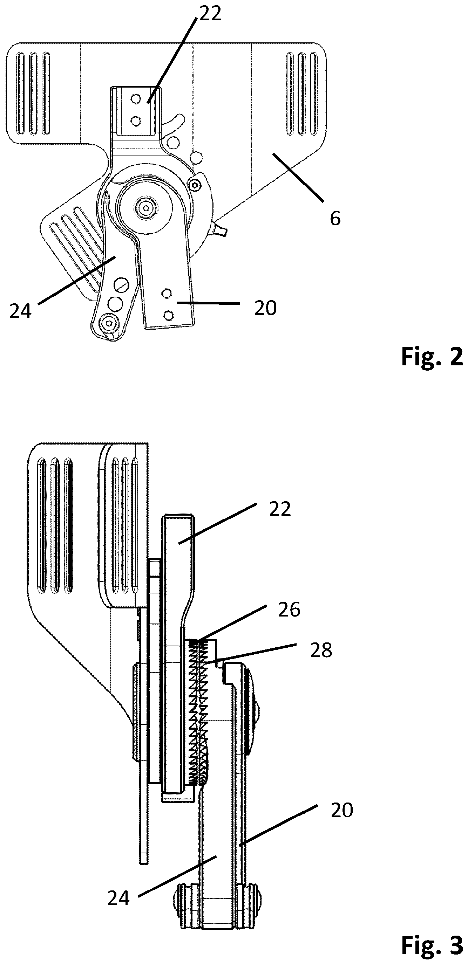

Figur 2 zeigt das Beckenelement 6 in einer vergrößerten Darstellung. Man erkennt ein erstes Verbindungselement 20, an dem die erste Schiene 12 des Oberschenkelelementes 2 anzuordnen ist, und ein zweites Verbindungselement 22, an dem die zweite Schiene 16 des Oberkörperelementes 4 angeordnet wird. Ein Hebel 24 ist vorhanden, an dem der mechanische Energiespeicher 18 positioniert wird.

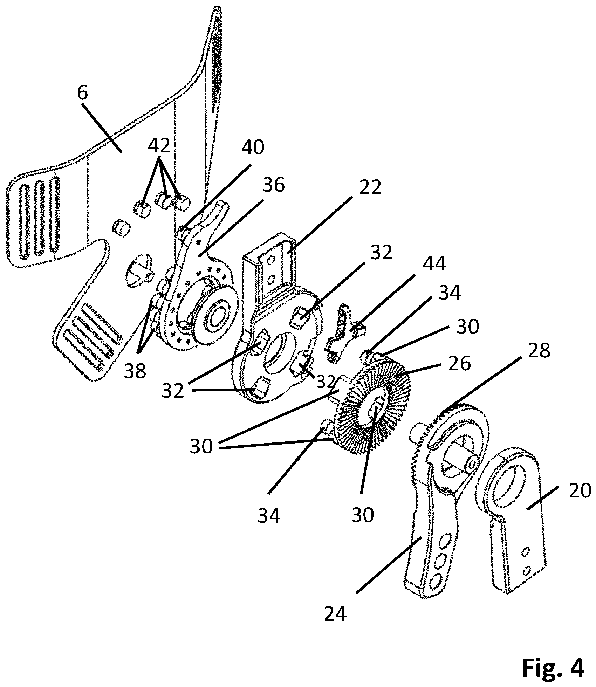

Figur 3 zeigt die Vorrichtung aus Figur 2 in einer Seitenansicht. Am zweiten Verbindungselement 22, das Teil des Oberkörperelementes 4 ist, befindet sich ein erstes Kraftübertragungselement 26, das im gezeigten Ausführungsbeispiel als Stirnzahnrad ausgebildet ist. Ein korrespondierendes zweites Kraftübertragungselement 28 ist am Hebel 24 positioniert, der Teil des Oberschenkelelementes 2 ist. Aus der Explosionsdarstellung in Figur 4 kann die Funktionsweise erkannt werden. Am Hebel 24 befindet sich das zweite Kraftübertragungselement 28. Am zweiten Verbindungselement 22 befindet sich als separates Bauteil das erste Kraftübertragungselement 26. Es verfügt über vier Vorsprünge 30, die in vier dafür vorgesehene Öffnungen 32 am zweiten Verbindungselement 22 eingreifen. Man erkennt in Figur 4, dass an zwei der Vorsprüngen 30 jeweils ein Magnet 34 angeordnet ist, der im montierten Zustand durch die jeweiligen Öffnungen 32 hindurchragt. An dem eigentlichen Deckenelement ist drehfest eine Verschiebeeinrichtung 36 angeordnet, die ebenfalls über eine Reihe von Magneten 38 verfügt. Diese erstrecken sich im gezeigten Ausführungsbeispiel um den gesamten Umfang der Verschiebeeinrichtung 36 herum und wirken somit auf die Magnete 34 am ersten Kraftübertragungselement 26. Am in Figur 4 oberen Ende der Verschiebeeinrichtung 36 ist ein Positioniermagnet 40 dargestellt, der mit entsprechenden Gegenmagneten 42 zusammenwirkt, die am Beckenelement 6 angeordnet sind. Der Positioniermagnet 40 und die Gegenmagnete 42 sind so angeordnet, dass ungleichnamige Pole aufeinander zugerichtet sind. Im gezeigten Ausführungsbeispiel ist die Verschiebeeinrichtung 36 folglich in vier unterschiedlichen Positionen relativ zum Beckenelement 6 drehfest befestigbar.

Wird nun das Oberkörperelement 4 und damit das zweite Verbindungselement 22 relativ zum Beckenelement 6 verdreht, verändert sich auch die Position der Magnete 34 relativ zu den Magneten 38. Diese sind so angeordnet, dass bei einem bestimmten Winkel, um den das Oberkörperelement 4 relativ zum Beckenelement 6 verdreht wird, die Polung der Magnete 38 wechselt, so dass bis zu diesem Winkel zwischen den Magneten 34 und 38 beispielsweise eine anziehende Kraft und ab diesem Winkel eine abstoßende Kraft wirkt. In diesem Moment, in dem aus einer anziehenden Kraft eine abstoßende Kraft wird, wird das erste Kraftübertragungselement 26 aus der in Figur 3 gezeigten Position verschoben und in Eingriff mit dem zweiten Kraftübertragungselement 28 gebracht.

Durch eine Arretiervorrichtung 44, die in Umfangsrichtung verschoben werden kann, lässt sich die Position des ersten Kraftübertragungselementes 26 relativ zum zweiten Kraftübertragungselement 28 fixieren, so dass es nicht mehr zu einem Verschieben eines der beiden Kraftübertragungselemente kommen kann. Dadurch wird entweder verhindert, dass die beiden Kraftübertragungselemente 26, 28 in Eingriff oder außer Eingriff gebracht werden können.

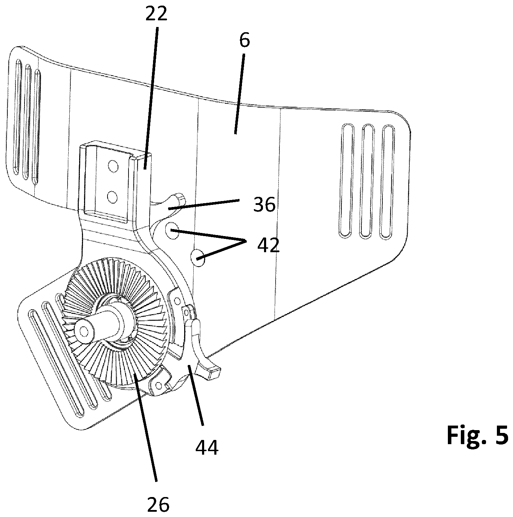

Figur 5 zeigt einen Teil der in Figur 4 gezeigten Elemente in einer teilweisen montierten Darstellung. Am Beckenelement 6 befindet sich die Verschiebeeinrichtung 36. Zu sehen sind zudem zwei Gegenmagnete 42 sowie das erste Kraftübertragungselement 26. Über die Arretiervorrichtung 44 kann das erste Kraftübertragungselement 26 relativ zum zweiten Verbindungselement 22 fixiert werden.

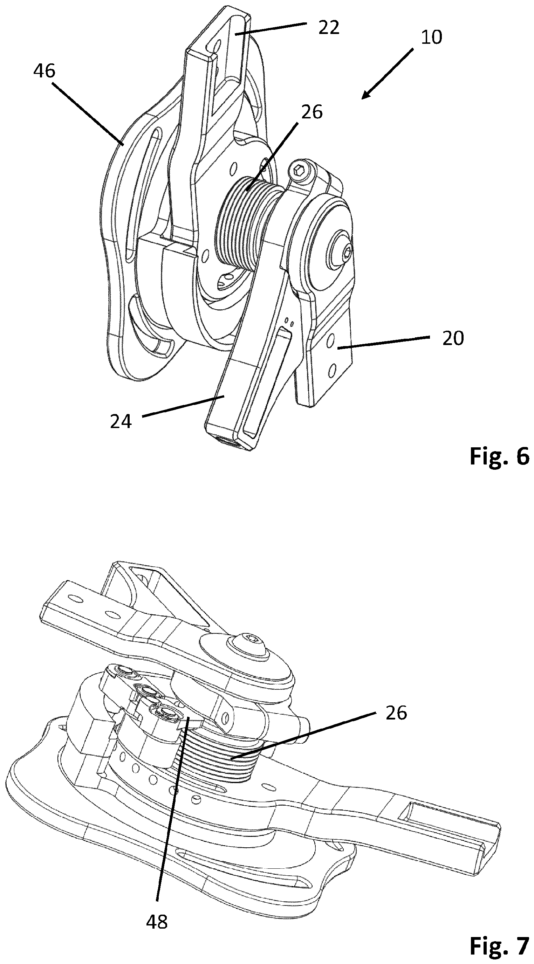

Figur 6 zeigt eine Gelenkeinrichtung 10 für eine orthopädietechnische Einrichtung gemäß einem weiteren Ausführungsbeispiel der vorliegenden Erfindung. Auch sie verfügt über ein zweites Verbindungselement 22, ein erstes Verbindungselement 20 sowie einen Hebel 24 und wird über eine Befestigungsplatte 46 an einem nicht dargestellten Beckenelement 6 positioniert. Das erste Kraftübertragungselement 26 ist im gezeigten Ausführungsbeispiel als Schlingfeder ausgebildet. Das zweite Kraftübertragungselement 28 ist in den Figuren 6 und 7 nicht dargestellt und ist als Stange oder Welle ausgebildet, die sich im Innern der Schlingfeder 26 erstreckt.

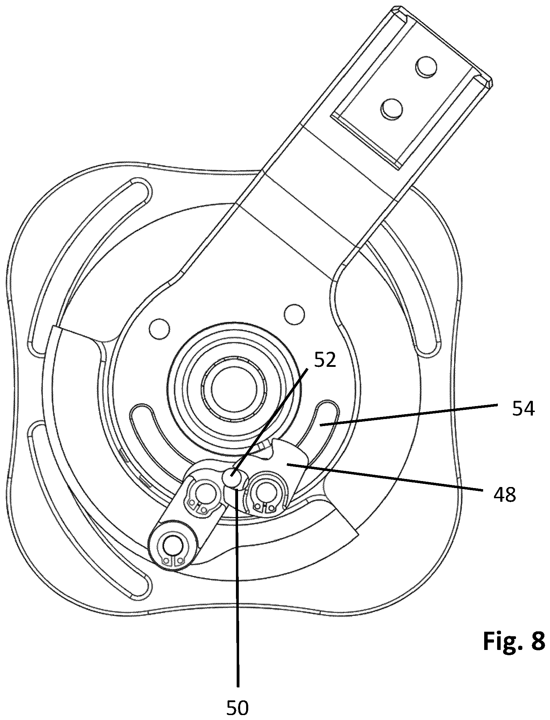

In Figur 7 ist die in Figur 6 gezeigte Vorrichtung aus einem anderen Blickwinkel darstellt. Man erkennt das erste Kraftübertragungselement 26. Am Oberschenkelelement 2 befindet sich ein Mitnehmer 48, der in der teilweise demontierten Darstellung von Figur 8 besser zu erkennen ist. Er verfügt über eine Ausnehmung 15, in die ein Pin 52 eingreift, wenn der Pin 52 relativ zur Ausnehmung 50 des Mitnehmers 48 bewegt wird. Dies geschieht entlang eines gebogenen Langloches 54. Nachdem der Pin 52 in Eingriff mit der Ausnehmung 50 gekommen ist, führt eine weitere Bewegung der beiden Bauteile dazu, dass auch die Schlingfeder so bewegt wird, dass sich ihr Querschnitt verkleinert. Dabei kommt es zu einer reibschlüssigen Verbindung zwischen dem als Schlingfeder ausgebildeten ersten Kraftübertragungselement und dem als Welle ausgebildeten zweiten Kraftübertragungselement 28, das sich im Innern der Schlingfeder 26 befindet.

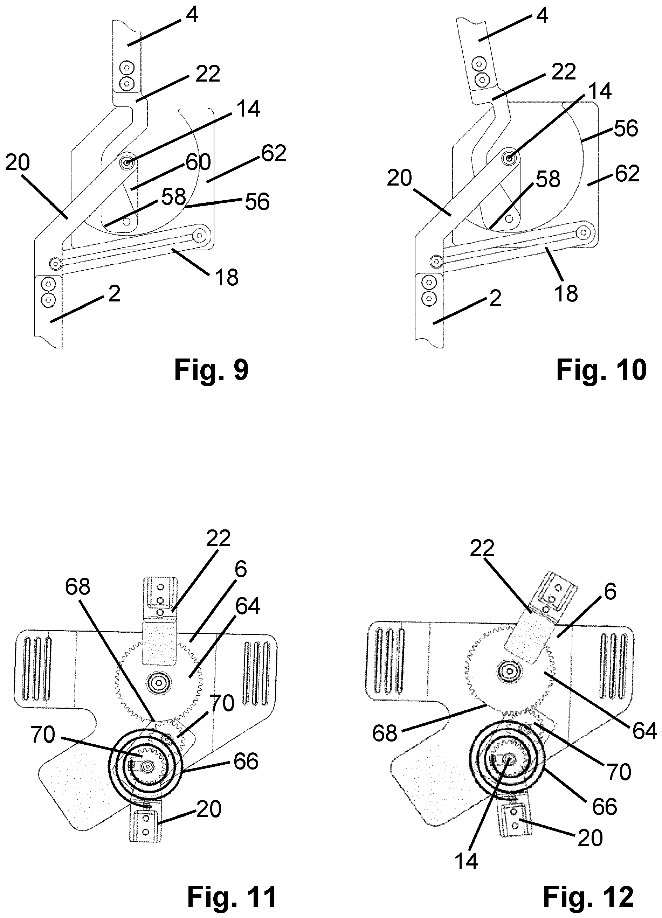

Figur 9 zeigt eine weitere Gelenkeinrichtung 10 in einer schematischen Darstellung. Am ersten Verbindungselement 20 ist ein Teil des Oberschenkelelementes 2 dargestellt. Am zweiten Verbindungselement 22 befindet sich ein Teil des Oberkörperelementes 4. Ein Gehäuse 62 ist dazwischen dargestellt. Der mechanische Energiespeicher 18 ist in Form eines elastischen Elementes zwischen dem ersten Verbindungselement 20 und dem Gehäuse 62 angeordnet, das drehbar an einem nicht dargestellten Beckenelement 6 angeordnet ist.

Am Gehäuse 62 befindet sich eine im gezeigten Ausführungsbeispiel kreisförmige Reibkontur 56. Das erste Verbindungselement 20 und damit das daran angeordnete Oberschenkelelement 2 sind um die erste Schwenkachse 14 schwenkbar am nicht dargestellten Beckenelement 6 angeordnet. Das zweite Verbindungselement 22 verfügt über einen Reibvorsprung 58 und ist über einen Hebel 60 um die gleiche Schwenkachse 14 schwenkbar am Beckenelement 6 angeordnet. Im gezeigten Ausführungsbeispiel bildet der Reibvorsprung 58 das erste Kraftübertragungselement 26 und die Reibkontur 56 das zweite Kraftübertragungselement 28.

Figur 9 zeigt die Situation, in der zwischen dem Reibvorsprung 58 und der Reibkontur 56 ein Spalt vorhanden ist, sodass es nicht zu einem Reibschluss zwischen den beiden kommt. Wird in diesem Zustand ein Oberschenkel, der mit dem Oberschenkelelement 2 verbunden ist, angehoben, wird er in der gezeigten Darstellung im Uhrzeigersinn um die erste Schwenkachse 14 verdreht. Durch den mechanischen Energiespeicher 18 wird auch das Gehäuse 62 um die Schwenkachse 14 verschwenkt. Da es keinen Reibschluss zwischen den beiden Kraftübertragungselementen 26, 28 gibt, wird der mechanische Energiespeicher 18 nicht aufgeladen.

Figur 10 zeigt die Situation, in der das Oberkörperelement 4 relativ zum Beckenelement 6, dass auch in Figur 10 nicht dargestellt ist, verschwenkt wurde, weil beispielsweise der Träger der orthopädietechnischen Einrichtung sich nach vorn gebeugt hat. Das Oberkörperelement 4 ist folglich relativ zum Beckenelement 6 gegen den Uhrzeigersinn verschwenkt worden. Dadurch kommt der Reibvorsprung 58 des zweiten Verbindungselementes 22 mit der Reibkontur 56 des Gehäuses 62 in Kontakt und wird gegen diese gedrückt, sodass es zu einem Reibschluss zwischen den beiden Elementen kommt. Wird in dieser Situation der Oberschenkel angehoben, also das Oberschenkelelement 2 im Uhrzeigersinn um die Schwenkachse 14 verschwenkt, kann das Gehäuse 62 aufgrund der reibschlüssigen Verbindung nicht mehr relativ zum Oberkörperelement 4 verschwenkt werden. Stattdessen wird der mechanische Energiespeicher 18 geladen und das elastische Element gedehnt. Dadurch wird eine Kraft erzeugt, die das Aufrichten des Trägers, also das Verschwenken des Oberschenkelelementes 2 entgegen dem Uhrzeigersinn, unterstützt.

Die Figuren 11 und 12 zeigen ein weiteres Ausführungsbeispiel. Am Beckenelement 6 ist über ein drehbares Zahnrad 64 das zweite Verbindungselement 22 angeordnet. Am ersten Verbindungselement 20, an dem das in den Figuren 11 und 12 nicht gezeigte Oberschenkelelement 2 angeordnet werden kann, ist eine Spiralfeder 66 angeordnet. Man erkennt, dass das Zahnrad 64 einen zahnlosen Bereich 68 aufweist. In Figur 11 ist die Situation dargestellt, in der das Oberschenkelelement 12 und das erste Verbindungselement 20 gegen den Uhrzeigersinn verschwenkbar ist, ohne dass die Spiralfeder 66 energetisch aufgeladen wird. In diesem Fall werden die kleinen Zahnräder 70 gedreht. Da das obere der kleinen Zahnräder 70 mit seinen Zähnen in dem zahnlosen Bereich 68 des Zahnrades 64 liegt, befindet sich dieses kleine Zahnrad 70 nicht mit den Zahnrad 64 in Eingriff, sodass die Rotation der kleinen Zahnräder 70 möglich ist, ohne die Spiralfeder 66 zu laden.

Figur 12 zeigt eine andere Situation, in der das Oberkörperelement 4, dass an dem zweiten Verbindungselement 22 anzuordnen ist, relativ zum Hüftelement 6 verschwenkt wurde. Dadurch wird auch das Zahnrad 64 um seine Rotationsachse gedreht, sodass nun nicht mehr der zahnlosen Bereich 68 sondern ein mit Zähnen versehener Bereich mit dem oberen kleinen Zahnrad 70 in Eingriff ist. Wird in dieser Situation das Oberschenkelelement 2 mit dem ersten Verbindungselement 20 verschwenkt und um die erste Schwenkachse 14 gegen den Uhrzeigersinn verschwenkt, wird die Spiralfeder 66 aufgeladen.

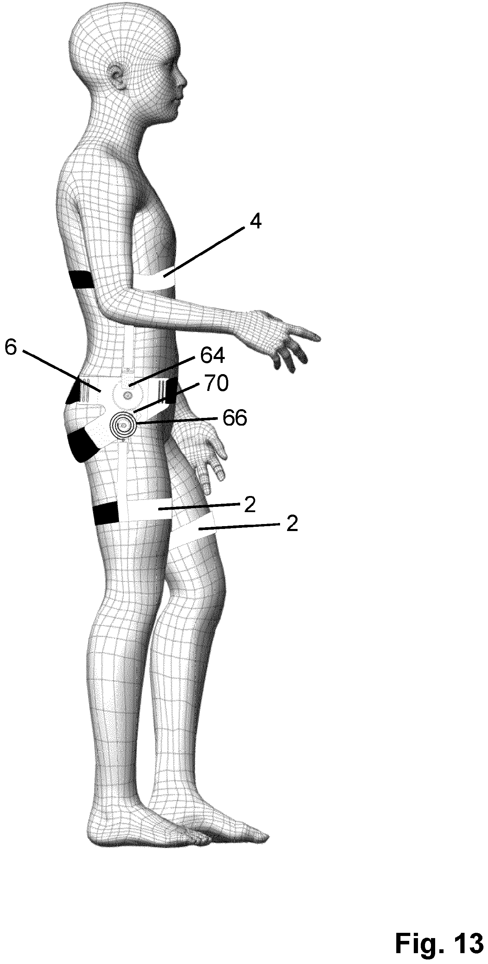

Die Figuren 13 bis 15 zeigen die Vorrichtung mit den Elementen aus den Figuren 11 und 12 im angelegten Zustand in unterschiedlichen Positionen. Man erkennt jeweils ein Oberschenkelelement 2, ein Oberkörperelement 4 sowie das Hüftelement 6, an dem insbesondere die Spiralfeder 66 und das Zahnrad 64 deutlich zu erkennen sind. Figur 13 zeigt den Träger der Vorrichtung in einer aufrechten Position. Das Zahnrad 64 ist aufgrund seines zahnlosen Bereichs 68 nicht mit dem kleinen Zahnrad 70 in Eingriff, sodass die Spiralfeder 66 unabhängig von der Position des Oberschenkelelements 2 nicht gespannt wird.

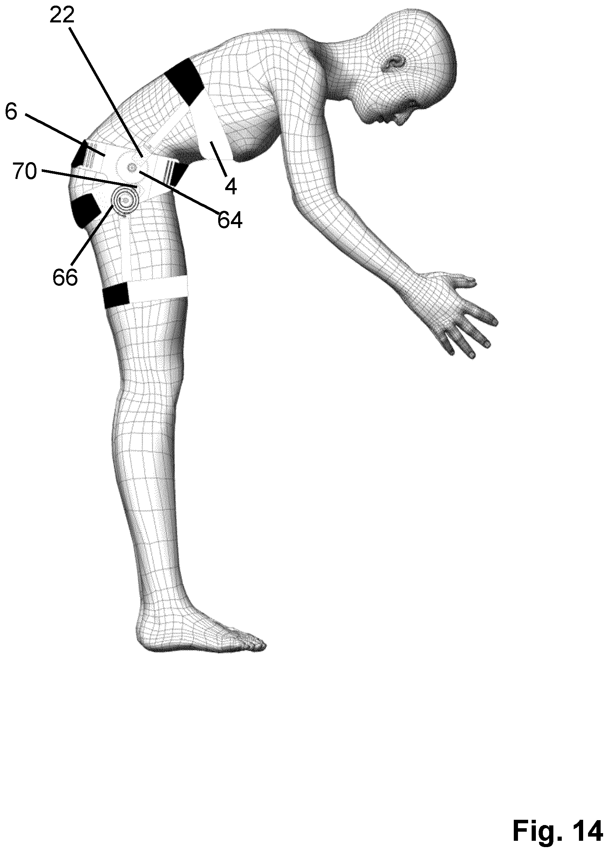

Figur 14 zeigt die Situation, in der der Träger der Vorrichtung seinen Oberkörper nach vorn gebeugt. Dadurch wird das Oberkörperelement 4 mit dem zweiten Verbindungselement 22 relativ zum Beckenelement 6 verschwenkt. Das Zahnrad 64 wird um seine Schwenkachse herum gedreht und kommt nun mit den Zähnen des kleinen Zahnrades 70 in Eingriff, sodass die Spiralfeder 66 ab diesem Moment gespannt wird. Selbstverständlich muss zum Spannen der Spiralfeder 66 nicht zwangsläufig der Oberschenkel bewegt werden. Es ist auch möglich, den Oberkörper weiter abzusenken, um den mechanischen Energiespeicher 18, im gezeigten Ausführungsbeispiel also die Spiralfeder 66, zu spannen.

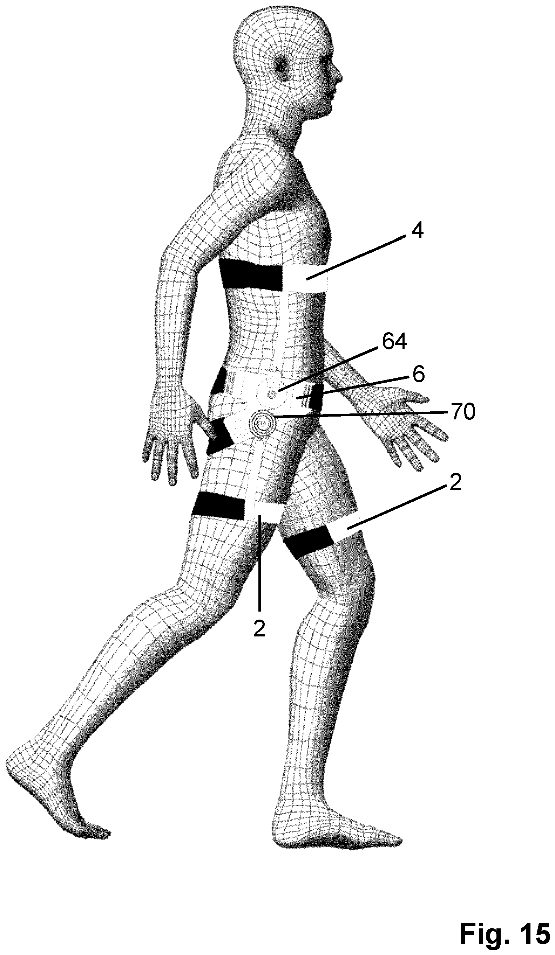

Figur 15 zeigt die Person beim Gehen. Auch hier ist der Oberkörper und das Oberkörperelement 4 nicht relativ zum Hüftgelenk 6 verschwenkt, sodass das Zahnrad 64 nicht mit dem oberen kleinen Zahnrad 70 in Eingriff ist und daher die Spiralfeder 66 nicht gespannt wird.

### Bezugszeichenliste:

- 2: Oberschenkelelement
- 4: Oberkörperelement
- 6: Beckenelement
- 8: Gurt
- 10: Gelenkeinrichtung
- 12: erste Schiene
- 14: erste Schwenkachse
- 16: zweite Schiene
- 18: mechanischer Energiespeicher
- 20: erstes Verbindungselement
- 22: zweites Verbindungselement
- 24: Hebel
- 26: erstes Kraftübertragungselement
- 28: zweites Kraftübertragungselement
- 30: Vorsprung
- 32: Öffnung
- 34: Magnet
- 36: Verschiebeeinrichtung
- 38: Magnet
- 40: Positioniermagnet
- 42: Gegenmagnet
- 44: Arretiervorrichtung
- 46: Befestigungsplatte
- 48: Mitnehmer
- 50: Ausnehmung
- 52: Pin
- 54: Langloch
- 56: Reibkontur
- 58: Reibvorsprung
- 60: Hebel
- 62: Gehäuse
- 64: Zahnrad
- 66: Spiralfeder
- 68: zahnloser Bereich
- 70: kleines Zahnrad

## Patentansprüche

1. Orthopädietechnische Einrichtung zum Unterstützen eines unteren Rückens eines Benutzers, wobei die Einrichtung
- einen mechanischen Energiespeicher (18),
- ein Oberkörperelement (4) mit einem ersten Kraftübertragungselement (26) und
- ein Oberschenkelelement (2) mit einem zweiten Kraftübertragungselement (28) aufweist,
wobei
• der mechanische Energiespeicher (18) aufladbar und entladbar ist, indem das Oberschenkelelement (2) relativ zu dem Oberkörperelement (4) verschwenkt wird, wenn das erste Kraftübertragungselement (26) mit dem zweiten Kraftübertragungselement (28) in Eingriff ist, und die Einrichtung ein Beckenelement (6) aufweist, wobei
• das Oberschenkelelement (2) um eine erste Schwenkachse (14) schwenkbar an dem Beckenelement (6) angeordnet ist und
• das Oberkörperelement (4) relativ zu dem Beckenelement (6) bewegbar angeordnet ist,
• das erste Kraftübertragungselement (26) mit dem zweiten Kraftübertragungselement (28) in Eingriff und außer Eingriff bringbar ist, indem das Oberkörperelement (4) relativ zu dem Beckenelement (6) bewegt wird.

2. Orthopädietechnische Einrichtung nach Anspruch 1, **dadurch gekennzeichnet, dass** das erste Kraftübertragungselement (26) ein Zahnrad aufweist, das exzentrisch um eine zweite Schwenkachse schwenkbar an dem Beckenelement (6) angeordnet ist.

3. Orthopädietechnische Einrichtung nach Anspruch 2, **dadurch gekennzeichnet, dass** das Oberkörperelement (4) derart mit dem ersten Kraftübertragungselement (26) verbunden ist, dass das erste Kraftübertragungselement (26) um die zweite Schwenkachse verschwenkt wird, wenn das Oberkörperelement (4) relativ zu dem Beckenelement (6) bewegt wird.

4. Orthopädietechnische Einrichtung nach Anspruch 2 oder 3, **dadurch gekennzeichnet, dass** das Oberkörperelement (4) und das erste Kraftübertragungselement (26) zueinander korrespondierende Verbindungselemente (20, 22) aufweisen, so dass das Oberkörperelement (4) in mehreren Positionen mit dem ersten Kraftübertragungselement (26) verbindbar ist.

5. Orthopädietechnische Einrichtung nach Anspruch 1, **dadurch gekennzeichnet, dass** die Einrichtung eine Verschiebeeinrichtung (36) aufweist, die eingerichtet ist, das erste Kraftübertragungselement (26) und/oder das zweite Kraftübertragungselement (28) aufeinander zu zu bewegen, wenn das Oberkörperelement (4) zu dem Beckenelement (6) einen Winkel einnimmt, der kleiner als ein vorbestimmter Grenzwinkel ist.

6. Orthopädietechnische Einrichtung nach Anspruch 5, **dadurch gekennzeichnet, dass** die Verschiebeeinrichtung (36) eingerichtet ist, das erste Kraftübertragungselement (26) und/oder das zweite Kraftübertragungselement (28) voneinander weg zu bewegen, wenn das Oberkörperelement (4) zu dem Beckenelement (6) einen Winkel einnimmt, der größer als der vorbestimmte Grenzwinkel ist

7. Orthopädietechnische Einrichtung nach Anspruch 5 oder 6, **dadurch gekennzeichnet, dass** an dem Beckenelement (6) oder dem Oberschenkelelement (2) wenigstens zwei Magnete (34) und an dem jeweils anderen wenigstens ein Magnet (34) derart angeordnet sind, dass sie eine Kraft aufeinander ausüben, deren Richtung wechselt, wenn der Winkel beim Bewegen des Oberkörperelementes (4) relativ zu dem Beckenelement (6) den vorbestimmten Grenzwinkel passiert.

8. Orthopädietechnische Einrichtung nach einem der vorstehenden Ansprüche, **dadurch gekennzeichnet, dass** die Einrichtung in einen aktiven Zustand, in dem das erste Kraftübertragungselement (26) mit dem zweiten Kraftübertragungselement (28) in Eingriff und außer Eingriff bringbar ist, indem das Oberkörperelement (4) relativ zu dem Beckenelement (6) bewegt wird, und in einen passiven Zustand bringbar ist.

9. Orthopädietechnische Einrichtung nach Anspruch 8, **dadurch gekennzeichnet, dass** die Einrichtung wenigstens ein Betätigungselement aufweist, durch dessen Betätigung die Einrichtung von dem aktiven Zustand in den passiven Zustand und/oder umgekehrt bringbar ist.

10. Orthopädietechnische Einrichtung nach einem der vorstehenden Ansprüche, **dadurch gekennzeichnet, dass** der mechanische Energiespeicher (18) wenigstens eine Feder, vorzugsweise wenigstens eine Spiralfeder, aufweist.

## Claims

1. An orthopedic device for supporting a lower back of a user, wherein the device comprises
- a mechanical energy store (18),
- an upper body element (4) with a first force transmission element (26) and
- an upper leg element (2) with a second force transmission element (28), wherein
• the mechanical energy store (18) can be charged and discharged by swiveling the upper leg element (2) relative to the upper body element (4) when the first force transmission element (26) is engaged with the second force transmission element (28), and
the device comprises a pelvic element (6), wherein
• the upper leg element (2) is arranged on the pelvic element (6) such that it can be swiveled about a first swivel axis (14),
• the upper body element (4) is movably arranged relative to the pelvic element (6),
• the first force transmission element (26) can be engaged and disengaged with the second force transmission element (28) by moving the upper body element (4) relative to the pelvic element (6).

2. The orthopedic device according to claim 1, **characterized in that** the first force transmission element (26) comprises a gearwheel that is eccentrically arranged on the pelvic element (6) such that it can be swiveled about a second swivel axis.

3. The orthopedic device according to claim 2, **characterized in that** the upper body element (4) is connected to the first force transmission element (26) such that the first force transmission element (26) is swiveled about the second swivel axis when the upper body element (4) is moved relative to the pelvic element (6).

4. The orthopedic device according to claim 2 or 3, **characterized in that** the upper body element (4) and the first force transmission element (26) comprise connection elements (20, 22) that correspond with one another, so that the upper body element (4) can be connected to the first force transmission element (26) in several positions.

5. The orthopedic device according to claim 1, **characterized in that** the device has a displacement device (36) that is configured to move the first force transmission element (26) and/or the second force transmission element (28) towards one another when the upper body element (4) assumes an angle in relation to the pelvic element (6) that is smaller than a pre-determined threshold angle.

6. The orthopedic device according to claim 5, **characterized in that** the displacement device (36) is configured to move the first force transmission element (26) and/or the second force transmission element (28) away from one another when the upper body element (4) assumes an angle in relation to the pelvic element (6) that is greater than the pre-determined threshold angle.

7. The orthopedic device according to claim 5 or 6, **characterized in that** at least two magnets (34) are arranged on the pelvic element (6) or the upper leg element (2) and at least one magnet (34) is arranged on the respective other element in such a way that they exert a force on one another, the direction of which changes when, during a movement of the upper body element (4) relative to the pelvic element (6), the angle passes the pre-determined threshold angle.

8. The orthopedic device according to one of the above claims, **characterized in that** the device can be brought into an active state, in which the first force transmission element (26) can be engaged and disengaged with the second force transmission element (28) by moving the upper body element (4) relative to the pelvic element (6), and into a passive state.

9. The orthopedic device according to claim 8, **characterized in that** the device comprises at least one activation element, the activation of which allows for the device to be brought from the active state into the passive state, and/or vice-versa.

10. The orthopedic device according to one of the above claims, **characterized in that** the mechanical energy store (18) has at least one spring, preferably at least one spiral spring.

## Revendications

1. Dispositif orthopédique pour soutenir le bas du dos d'un utilisateur, le dispositif comprenant
- un accumulateur d'énergie mécanique (18),
- un élément de torse (4) avec un premier élément de transmission de force (26), et
- un élément de cuisse (2) avec un deuxième élément de transmission de force (28),
dans lequel
• l'accumulateur d'énergie mécanique (18) peut être chargé et déchargé par pivotement de l'élément de cuisse (2) par rapport à l'élément de torse (4), lorsque le premier élément de transmission de force (26) est en prise avec le deuxième élément de transmission de force (28), et
le dispositif comprend un élément de bassin (6),
• l'élément de cuisse (2) est disposé sur l'élément de bassin (6) de manière à pouvoir pivoter autour d'un premier axe de pivotement (14), et
• l'élément de torse (4) étant disposé de manière mobile par rapport à l'élément de bassin (6),
• le premier élément de transmission de force (26) pouvant être mis en prise et hors de prise avec le deuxième élément de transmission de force (28) par déplacement de l'élément de torse (4) par rapport à l'élément de bassin (6).

2. Dispositif orthopédique selon la revendication 1,
**caractérisé en ce que** le premier élément de transmission de force (26) présente une roue dentée qui est disposée excentriquement sur l'élément de bassin (6) de manière à pouvoir pivoter autour d'un deuxième axe de pivotement.

3. Dispositif orthopédique selon la revendication 2,
**caractérisé en ce que** l'élément de torse (4) est relié au premier élément de transmission de force (26) de telle sorte que le premier élément de transmission de force (26) est pivoté autour du deuxième axe de pivotement lorsque l'élément de torse (4) est déplacé par rapport à l'élément de bassin (6).

4. Dispositif orthopédique selon la revendication 2 ou 3,
**caractérisé en ce que** l'élément de torse (4) et le premier élément de transmission de force (26) présentent des éléments de liaison (20, 22) correspondant l'un à l'autre, de sorte que l'élément de torse (4) peut être relié au premier élément de transmission de force (26) dans plusieurs positions.

5. Dispositif orthopédique selon la revendication 1,
**caractérisé en ce que** le dispositif comporte un dispositif de translation (36) qui est conçu pour déplacer le premier élément de transmission de force (26) et/ou le deuxième élément de transmission de force (28) l'un vers l'autre, lorsque l'élément de torse (4) prend un angle par rapport à l'élément de bassin (6), qui est inférieur à un angle limite prédéterminé.

6. Dispositif orthopédique selon la revendication 5,
**caractérisé en ce que** le dispositif de translation (36) est conçu pour éloigner l'un de l'autre le premier élément de transmission de force (26) et/ou le deuxième élément de transmission de force (28), lorsque l'élément de torse (4) prend un angle par rapport à l'élément de bassin (6), qui est supérieur à l'angle limite prédéterminé.

7. Dispositif orthopédique selon la revendication 5 ou 6,
**caractérisé en ce qu'**au moins deux aimants (34) sont disposés sur l'élément de bassin (6) ou sur l'élément de cuisse (6), et au moins un aimant (34) est disposé sur l'autre parmi lesdits éléments, de manière à exercer mutuellement une force dont la direction change lorsque l'angle franchit l'angle limite prédéterminé lors du déplacement de l'élément de torse (4) par rapport à l'élément de bassin (6).

8. Dispositif orthopédique selon l'une des revendications précédentes, **caractérisé en ce que**
le dispositif peut être amené dans un état actif, dans lequel le premier élément de transmission de force (26) peut être mis en prise et hors de prise avec le deuxième élément de transmission de force (28) par déplacement de l'élément de torse (4) par rapport à l'élément de bassin (6), et être amené dans un état passif.

9. Dispositif orthopédique selon la revendication 8,
**caractérisé en ce que** le dispositif comprend au moins un élément d'actionnement, par l'actionnement duquel le dispositif peut être amené de l'état actif à l'état passif et/ou inversement.

10. Dispositif orthopédique selon l'une des revendications précédentes, **caractérisé en ce que** l'accumulateur d'énergie mécanique (18) comporte au moins un ressort, de préférence au moins un ressort en spirale.
